# EUROPEAN PATENT SPECIFICATION

(11) **EP 0 328 231 B1**
(45) Date of publication and mention of the grant of the patent: **06.07.1994**
(21) Application number: 89300106.5
(22) Date of filing: 06.01.1989
(51) Int. Cl.: C07K 17/00, G01N 33/531, G01N 33/577, G01N 33/563, G01N 33/546

(54) **Antibodies attached to solid surface**
Antikörper, gebunden an eine feste Oberfläche
Anticorps fixés à une surface solide

(30) Priority: 07.01.1988 GB 8800293
(43) Date of publication of application: 16.08.1989
(73) Proprietor: INTERNATIONAL INSTITUTE OF CELLULAR & MOLECULAR PATHOLOGY, B-1200 Brussels (BE)
(72) Inventor: Cambiaso, César Lorenzo, B-1950 Kraainem (BE)
(74) Representative: Wain, Christopher Paul

(56) References cited:
- WO-A-82/02773
- GB-A- 2 013 211
- US-A- 4 258 130
- JOURNAL OF IMMUNOLOGICAL METHODS, vol. 56, 1983, pages 235-243, Elsevier Biomedical Press, New York, US; E. LAMOYI et al.: "Preparation of F(ab')2 fragments from mouse IgG of various subclasses"

## Description

This invention relates to an immunochemical reagent comprising an antibody fragment attached to a solid surface.

It is known to attach antibodies to particulate material in suspension. Latex particles in the order of a few microns or sub-microns size are commercially available and widely used for this purpose. Monoclonal antibodies are preferred but the attachment of monoclonal antibodies to such particles tends to result in self-agglutination of the particles to a greater or lesser extent. This problem has never been solved, and as a result it has hitherto in general not been possible to use monoclonal antibodies immobilised on latex particles in immuno-assays involving agglutination.

In immuno-assays using antibodies, whether polyclonal or monoclonal, and whether in solution or attached to a solid support, there is often a problem of interference by other proteins which may be present in samples, for example serum samples, under assay. In particular, human serum contains rheumatoid factor (RF) and Clq (a component of complement), and both these substances bind with IgG antibody. Moreover, the amounts of RF and Clq in human sera can vary widely and it is usually necessary therefore to treat the sera (prior to the assay) to inactivate or remove endogenous RF and Clq. If this is not done, the assay results (particularly any quantitative result) may be significantly in error.

This problem is addressed in US-A-4397960, and solved by the expedient of using F(ab')₂ fragments rather than whole IgG antibodies. The fragments may be used in solution or attached to a solid surface. The immobilised reagent may be prepared, either by pepsic digestion of the antibodies in solution followed by attachment of the F(ab')₂ fragments to the solid surface; or by pepsic digestion of the whole antibodies after they have been attached, by means of reduced disulphide bridges, to the solid surface.

The liquid phase digestion technique of US-A-4397960 is satisfactory for polyclonal antibodies, such as may be obtained from rabbits, sheep, goats or horses. But it is well known in the art that mouse antibodies cannot be subjected to pepsic digestion in the liquid phase without risk of substantially destroying the immunoreactivity of any resulting F(ab')₂ fragment (E. Lamoyi and A. Nisonoff, Journal of Immunological Methods, 56 (1983), 235-243). The loss of immunoreactivity varies according to the antibody under investigation, but it is not possible to choose digestion conditions which avoid this loss. Indeed, although F(ab')₂ fragments of mouse antibodies, particularly monoclonal antibodies, have long been recognised as desirable reagents for immuno-assays, it has been generally accepted that such reagents with satisfactory immunoreactivity are difficult to obtain.

We have now made the surprising discovery that mouse and rat antibodies, when attached to a solid surface, can be subjected to pepsic digestion without substantial loss of immunoreactivity.

According to the present invention, there is provided an immunochemical reagent which comprises F(ab')₂ fragments of IgG antibodies attached to a solid surface, the reagent having been prepared by attaching IgG antibody to said solid surface and subjecting it, while so attached, to digestion with an enzyme to remove Fc fragments and leave F(ab')₂ fragments attached to the solid surface; characterised in that the antibodies are mouse or rat antibodies and the attached fragments have at least 70% of the immunoreactivity of the respective free whole antibody.

The invention also provides a method of making an immunochemical reagent, which comprises attaching IgG antibody to a solid surface, and subjecting the attached antibody to digestion with an enzyme to remove Fc fragments and leave F(ab')₂ fragments of the antibody attached to the solid surface, characterised in that the antibody is mouse or rat antibody, and the attached fragments have at least 70% of the immunoreactivity of the respective free whole antibody.

The invention is specific to mouse and rat antibodies, of the IgG class, and is of particular advantage when monoclonal antibodies are used. The covalent attachment of such antibodies to solid surfaces is well known. This is usually effected by providing a reactive coating on the surface, and then chemically linking or adsorbing the antibody thereon. It is also possible in certain circumstances to bind the antibody directly to the surface with no intervening coating. The manner of attachment of the antibody to the solid surface does not form part of the present invention and is well known in the art, and will not be further described here.

The nature of the solid surface is not critical. It may for example be in the form of a sheet or hollow tube or dipstick, or as a coating on the surface of the assay vessel, or it may be particulate material which may be magnetically attractable. Particulate material of particular interest is of microscopic or sub-microscopic size, i.e. generally smaller than 15 microns and most usually of the order of a few microns or submicron in size. Latex particles of such sizes are commercially available.

After attachment to the solid surface, the antibodies are subjected to a controlled enzyme digestion. This has the effect of removing Fc fragments and leaving immunoreactive F(ab')₂ fragments on the latex surface. It is quite likely that covalent coupling of the antibodies to the solid surface stabilises the F(ab')₂ structure and renders it more resistant to the digestion. The digestion should not be so severe that the immunoreactivity of the fragments is substantially reduced. On the other hand, the digestion should be sufficiently severe that the particles after digestion have no substantial tendency to self-agglutination. This is a balance between conflicting requirements, and one on which it is not appropriate to place precise limits. As a general rule, the immunoreactivity of the product after enzyme digestion should be at least 70% of that of the whole antibody prior to digestion.

Digestion conditions required to achieve these aims vary from antibody to antibody, and are quite readily determined by routine experiment. The digestion enzyme is preferably pepsin, though other enzymes are available and known to those skilled in the art. Typical digestion conditions may involve incubation of enzyme at a pepsin/antibody ratio of 1 to 15% for a time of from 1 minute to 48 hours, at a pH of around 4 to 4.5 and a temperature around 37°C.

The following examples illustrate the invention.

### EXAMPLE 1

A total of twenty one different antibodies were attached to carboxylated latex particles by covalent coupling in a conventional manner. While so attached, the antibodies were subjected to pepsin digestion under the conditions set out in Table 1 below. The pepsin digestion removed any tendency of the coated particles to pre-agglutinate. And the immunoreactivity after digestion was in all cases at least as good as before digestion, and hence is given in Table 1 as 100%.

For purposes of comparison, the same antibodies were digested in the liquid phase, by incubation to form the 37°C, pH 4.2, with a pepsin/protein ratio of 3%. The incubation time was variable:

| Subclass | Incubation time |
|---|---|
| IgG1 | 8 h. |
| IgG2a | 8 h. |
| IgG2b | 30 min. |
| IgG3 | 15 min. |

The activity recovery after this liquid phase digestion was measured by comparing the inhibiting power of the digested IgG and the undigested IgG. The agglutination of antibody-coated latex by albumin-DNP conjugate (DNP is dinitrophenol) was inhibited by digested and non-digested IgG. The percentage of inhibition was considered as 100 for intact IgG. Details and results are set out in the following Table 1.

**Table 1**

| Pepsin digestion of latex coated with different subclasses of monoclonal antibodies | | | | | | |
|---|---|---|---|---|---|---|
| Monoclonal | Subclass | Treatment | | | Activity recovery after digestion of latex-IgG (%) | Activity recovery after digestion of soluble-IgG (%) |
| | | % pepsin | pH | Incubation at 37°C | | |
| Anti-DNP | | | | | | |
| A | IgG1 | 6 | 4.2 | 20 h. | 100 | 58 |
| B | IgG1 | 6 | 4.2 | 20 h. | 100 | 73 |
| C | IgG1 | 6 | 4.4 | 20 h. | 100 | 92 |
| D | IgG1 | 6 | 4.4 | 20 h. | 100 | 100 |
| E | IgG2a | 6 | 4.2 | 1 h. | 100 | 85 |
| F | IgG2a | 6 | 4.2 | 2 h. | 100 | 60 |
| G | IgG2a | 6 | 4.2 | 20 h. | 100 | 86 |
| H | IgG2a | 6 | 4.4 | 30 min. | 100 | 40 |
| I | IgG2a | 6 | 4.2 | 2 h. | 100 | 52 |
| J | IgG2b | 6 | 4.4 | 10 min. | 100 | 31 |
| K | IgG2b | 6 | 4.2 | 30 min. | 100 | 56 |
| L | IgG2b | 6 | 4.2 | 10 min. | 100 | 72 |
| M | IgG2b | 6 | 4.4 | 10 min. | 100 | 0 |
| N | IgG2b | 6 | 4.2 | 30 min. | 100 | 56 |
| O | IgG3 | 6 | 4.2 | 1 h. | 100 | 30 |
| P | IgG3 | 6 | 4.4 | 10 min. | 100 | 0 |
| Q | IgG3 | 6 | 4.2 | 1 h. | 100 | 70 |
| R | IgG3 | 6 | 4.2 | 1 h. | 100 | 18 |

| Anti-brucella | | | | | | |
|---|---|---|---|---|---|---|
| S | IgG2a | 6 | 4.2 | 20 h. | 100 | n.d. |
| T | IgG1 | 6 | 4.2 | 20 h. | 100 | n.d. |
| U | IgG3 | 6 | 4.4 | 1 h. | 100 | n.d. |

Inspection of the results in the right hand column of the table shows that liquid phase digestion of the monoclonal anti-DNP antibodies resulted in very substantial loss of immunoreactivity. For the anti-brucella antibodies, comparable figures were not obtained, but they were considerably less than 100%.

### EXAMPLE 2

Diluted particles, coated with different undigested and digested antibodies as in Example 1, were counted in a particle counting system of the IMPACT (trademark) machine. The peak height was set at 100. The resulting gain setting is related to the number of monomeric latex particles. The higher the gain setting, the lower is the number of monomeric particles, i.e. the greater is the tendency to self-agglutination. The results are set out in Table 2 below. In all cases, it can be seen that digestion of the latex antibody reduced the tendency to self-agglutination. Self-agglutination of the latex-anti-brucella antibodies was not measured, but would certainly have shown improvements of the same order.

**Table 2**

| Improvement of Selfagglutination | | | |
|---|---|---|---|
| Monoclonal | Subclass | Gain | |
| | | Undigested Lx | Digested Lx |
| A | IgG1 | 1.72 | 1.5 |
| B | IgG1 | 2.5 | 1.49 |
| C | IgG1 | 1.5 | 1.49 |
| D | IgG1 | 2.5 | 1.6 |
| E | IgG2a | 3.08 | 1.73 |
| F | IgG2a | 2.18 | 1.3 |
| G | IgG2a | 10 | 2.76 |
| H | IgG2a | 3.12 | 1.75 |
| I | IgG2a | 5.7 | 1.86 |
| J | IgG2b | 2.89 | 2.47 |
| K | IgG2b | 7.5 | 3.26 |
| L | IgG2b | 6.9 | 2.82 |
| M | IgG2b | 2.97 | 2.41 |
| N | IgG2b | 3.05 | 1.92 |
| O | IgG3 | 5.3 | 1.8 |
| P | IgG3 | 2.54 | 1.78 |
| Q | IgG3 | 1.81 | 1.6 |
| R | IgG3 | 1.68 | 1.5 |

### EXAMPLE 3

The results in this Example were obtained with latex C or Example 1. The other latexes of Example 1 are known to give rise to similar results.

30 micro litres of a suspension of digested and non-digested latex C were incubated under agitation at 37°C with 30 micro litres of dithiothreitol-treated sera (dilution of 1:5). No specific agglutination with the sera was expected, since the latex was coated with a monoclonal anti-DNP antibody. The experiment is designed to check for variations in non-specific agglutination, perhaps caused by unknown species in different sera. A total of 26 different sera from different sources, designated a to z in table 3 below, were used for this purpose.

Agglutination was again measured using the particle counting system of the IMPACT machine. The peak height was set at or a little below 100. Peak heights are reported in Table 3 below and are related to numbers of monomeric (unagglutinated) particles.

The right hand column in the Table shows that the number of monomeric particles of digested latex does not vary a lot when mixed with 26 different sera. On the contrary, when undigested latex was mixed with the same 26 sera, variations in the peak heights occured, indicating non-specific agglutination in the majority of the sera. This serum to serum variation of peak height is statistically expressed in the form of a coefficient of variation.

| Effect of digestion of Latex-IgG on serum interferences | | |
|---|---|---|
| Serum | Peak height | |
| | Undigested Lx | Digested Lx |
| a | 92 | 95 |
| b | 88 | 95 |
| c | 88 | 94 |
| d | 82 | 94 |
| e | 86 | 94 |
| f | 92 | 94 |
| g | 85 | 95 |
| h | 90 | 94 |
| i | 88 | 95 |
| j | 84 | 95 |
| k | 82 | 94 |
| l | 84 | 94 |
| m | 86 | 94 |
| n | 80 | 94 |
| o | 84 | 95 |
| p | 88 | 95 |
| q | 90 | 95 |
| r | 90 | 95 |
| s | 88 | 95 |
| t | 90 | 94 |
| u | 90 | 95 |
| v | 88 | 95 |
| w | 88 | 95 |
| x | 94 | 96 |
| y | 90 | 95 |
| z | 88 | 96 |
| n = 26 | X : 87 | 94 |
| | Sd : 3.39 | 0.6 |
| | CV : 3.9% | 0.65% |

## Claims

1. An immunochemical reagent which comprises F(ab')₂ fragments of IgG antibodies attached to a solid surface, the reagent having been prepared by attaching the IgG antibody to said solid surface and subjecting it, while so attached, to digestion with an enzyme to remove Fc fragments and leave F(ab')₂ fragments attached to the solid surface; characterised in that the antibodies are mouse or rat antibodies and the attached fragments have at least 70% of the immunoreactivity of the respective free whole antibody.

2. A reagent as claimed in claim 1, wherein the antibodies are monoclonal antibodies.

3. A reagent as claimed in claim 1 or claim 2, wherein the solid surface is particulate material in suspension.

4. A reagent as claimed in claim 3, wherein the particles have no substantial tendency to self-agglutination.

5. A method of making an immunochemical reagent, which comprises attaching IgG antibody to a solid surface, and subjecting the attached antibody to digestion with an enzyme to remove Fc fragments and leave F(ab')₂ fragments of the antibody attached to the solid surface, characterised in that the antibody is mouse or rat antibody, and the attached fragments have at least 70% of the immunoreactivity of the respective free whole antibody.

6. A method as claimed in claim 5, wherein the antibody is a monoclonal antibody.

7. A method as claimed in claim 5 or 6, wherein the solid surface is particulate material in suspension.

8. A method as claimed in claim 5, 6 or 7, wherein the proteolytic enzyme is pepsin.

9. A method as claimed in claim 7, wherein prior to digestion the antibody-coated particles tend to self-agglutinate, and the digestion is performed under conditions which substantially reduce the tendency to self-agglutination.

## Patentansprüche

1. Immunchemisches Reagenz, das F(ab')₂-Fragmente von an eine feste Oberfläche angehefteten IgG-Antikörpern enthält und dadurch hergestellt ist, daß der IgG-Antikörper an die feste Oberfläche angeheftet und in diesem angehefteten Zustand der Verdauung mit einem Enzym unterworfen wird, um Fc-Fragmente zu entfernen und die F(ab')₂-Fragmente an die feste Oberfläche angeheftet zu lassen,
**dadurch gekennzeichnet,**
daß die Antikörper Mäuse- oder Rattenantikörper sind und die angehefteten Fragmente mindestens 70% der Immunreaktivität des entsprechenden freien Gesamtantikörpers aufweisen.

2. Reagenz gemäß Anspruch 1, wobei die Antikörper monoclonale Antikörper sind.

3. Reagenz gemäß Anspruch 1 oder 2, wobei die feste Oberfläche durch teilchenförmiges Material in Suspension dargestellt wird.

4. Reagenz gemäß Anspruch 3, wobei die Teilchen im wesentlichen keine Neigung zur Selbstagglutinierung aufweisen.

5. Verfahren zur Herstellung eines immunchemischen Reagenzes, wobei man einen IgG-Antikörper an eine feste Oberfläche anheftet, und den angehefteten Antikörper der Verdauung durch ein Enzym unterwirft, um Fc-Fragmente zu entfernen und F(ab')₂-Fragmente des Antikörpers an die feste Oberfläche angeheftet läßt,
**dadurch gekennzeichnet,**
daß der Antikörper ein Mäuse- oder Rattenantikörper ist und die angehefteten Fragmente mindestens 70% der Immunreaktivität des entsprechenden freien Gesamtantikörpers aufweisen.

6. Verfahren gemäß Anspruch 5, wobei der Antikörper ein monoclonaler Antikörper ist.

7. Verfahren gemäß Anspruch 5 oder 6, wobei die feste Oberfläche von teilchenförmigem Material in Suspension dargestellt wird.

8. Verfahren gemäß Anspruch 5, 6 oder 7, wobei das proteolytische Enzym Pepsin ist.

9. Verfahren gemäß Anspruch 7, wobei die mit dem Antikörper überzogenen Teilchen vor der Verdauung zur Selbstagglutination neigen und die Verdauung unter Bedingungen durchgeführt wird, die die Neigung zur Selbstagglutination wesentlich verringern.

## Revendications

1. Réactif immunochimique comprenant des fragments F(ab')₂ d'anticorps IgG fixés à une surface solide, le réactif ayant été préparé en ce qu'on a fixé l'anticorps IgG à ladite surface solide et on l'a soumis, à l'état ainsi fixé, à une digestion avec une enzyme pour enlever des fragments Fc et laisser des fragments F(ab')₂ fixés à la surface solide, caractérisé en ce que les anticorps sont des anticorps de souris ou de rat, et les fragments fixés possèdent au moins 70 % de l'immunoréactivité de l'anticorps libre entier respectif.

2. Réactif selon la revendication 1, dans lequel les anticorps sont des anticorps monoclonaux.

3. Réactif selon la revendication 1 ou 2, dans lequel la surface solide est un matériau particulaire en suspension.

4. Réactif selon la revendication 3, dans lequel les particules n'ont pratiquement aucune tendance a l'auto-agglutination.

5. Procédé de préparation d'un réactif immunochimique, comprenant les étapes consistant à fixer un anticorps IgG à une surface solide et soumettre l'anticorps fixé à une digestion avec une enzyme pour enlever des fragments Fc et laisser des fragments F(ab')₂ fixés à la surface solide, caractérisé en ce que l'anticorps est un anticorps de souris ou de rat, et les fragments fixés possèdent au moins 70 % de l'immunoréactivité de l'anticorps libre entier respectif.

6. Procédé selon la revendication 5, dans lequel l'anticorps est un anticorps monoclonal.

7. Procédé selon la revendication 5 ou 6, dans lequel la surface solide est un matériau particulaire en suspension.

8. Procédé selon la revendication 5, 6 ou 7, dans lequel l'enzyme protéolytique est la pepsine.

9. Procédé selon la revendication 7, dans lequel, avant la digestion, les particules revêtues d'anticorps ont une tendance à l'auto-agglutination, et la digestion est effectuée dans conditions qui diminuent essentiellement leur tendance à l'auto-agglutination.
